# EUROPEAN PATENT APPLICATION

(11) **EP 3 287 917 A1**
(43) Date of publication of application: **28.02.2018**
(21) Application number: 17001425.2
(22) Date of filing: 23.08.2017
(51) Int. Cl.: G06F 19/00

(54) **DISTRIBUTED DATA GATHERING AND RECOMMENDATION IN PHYTOTHERAPY**

(30) Priority: 23.08.2016 US 201662378470 P
(71) Applicant: Clemmons, Michael S., Mill Valley, CA 94941 (US)
(72) Inventor: Clemmons, Michael S., Mill Valley, CA 94941 (US); Sweeny, Matt, Sacramento, CA 95833 (US)
(74) Representative: Müller-Boré & Partner Patentanwälte PartG mbB

(57) **Abstract**

Prediction of the physiological effect of phytotherapeutic products and the recommendation of phytotherapeutic products on the basis of physiological effects are provided. A first product profile includes a plurality of compound identifiers and a plurality of concentrations. Observational data is read regarding a plurality of subjects who have consumed a first product substantially conforming with the first product profile. From the observational data a first set of physiological effects is determined associated with the first product profile. In some embodiments, a second set of physiological effects associated with a second product profile is determined based on the first product profile, the second product profile, and the first set of physiological effects. In other embodiments, a second product profile associated with a second set of physiological effects is determined based on the first product profile, the first set of physiological effects, and the second set of physiological effects.

## Description

### CROSS-REFERENCE TO RELATED APPLICATIONS

This application claims the benefit of U.S. Provisional Application No. 62/378,470, filed August 23, 2016, which is hereby incorporated by reference in its entirety.

### FIELD

Embodiments of the present invention relate to distributed data gathering and recommendation in phytotherapy, and more specifically, to the prediction of the physiological effect of phytotherapeutic products and the recommendation of phytotherapeutic products on the basis of physiological effects.

### BACKGROUND

There is currently a lack of data available with respect to the effects of various plant-based medicines, remedies or health-promoting agents. In fact, even widely used remedies may not have undergone substantial clinical testing. The lack of data is particularly acute with respect to historically controlled substances, such as cannabis. The effects of plant-based products may vary significantly between strains, between preparations, and between delivery routes. Likewise, combination products may have effects on a user that differ from standalone products. Personalized medicine has been closely studied in the pharmaceutical setting, but has received little attention in other fields of medicine. The complicated interplay of strain, combination, preparation, delivery route, and user attributes has not been adequately analyzed.

Although many plant-based products are used, there has not been a mechanism for collecting data regarding their effects or a mechanism for determining appropriate products for use by a user to achieve a desired effect. Thus, there remains a need in the art for systems and methods for distributed data gathering and recommendation in phytotherapy.

### BRIEF SUMMARY

According to one aspect of the present invention, methods of and computer program products for distributed data gathering and recommendation in phytotherapy are provided. In certain embodiments, a first product profile is read. The first product profile includes a plurality of compound identifiers and a plurality of concentrations. Each of the plurality of compound identifiers identifies a compound. Each of the plurality of concentrations is associated with one of the plurality of compound identifiers. Observational data is read regarding a plurality of subjects. Each of the plurality of subjects has consumed a first product substantially conforming with the first product profile. Consumption may include any introduction of a product to a subject organism, whether through ingestion, inhalation, transdermal administration, or otherwise. From the observational data a first set of physiological effects is determined associated with the first product profile.

In some such embodiments, a second product profile is read. A second set of physiological effects associated with the second product profile is determined based on the first product profile, the second product profile, and the first set of physiological effects. Determining the second set of physiological effects may include training an artificial neural network using the first product profile and the first set of physiological effects and providing the second product profile to the artificial neural network.

In some such embodiments, a second set of physiological effects is read. A second product profile associated with the second set of physiological effects is determined based on the first product profile, the first set of physiological effects, and the second set of physiological effects. Determining the second product profile may include training an artificial neural network using the first product profile and the first set of physiological effects and providing the second set of physiological effects to the artificial neural network.

In some embodiments, characteristic data regarding the plurality of subjects is read. In some such embodiments, characteristic data regarding a target user is read. The second set of physiological effects associated with the second product profile may be determined based on the first product profile, the second product profile, the first set of physiological effects, and the characteristic data regarding the target user. Alternatively, a second product profile associated with the second set of physiological effects may be determined based on the first product profile, the first set of physiological effects, the second set of physiological effects, and the characteristic data regarding the target user.

In some embodiments, determining the second set of physiological effects comprises training an artificial neural network using the first product profile, the first set of physiological effects, and the characteristic data regarding the plurality of subjects and providing the second product profile and the characteristic data regarding the target user to the artificial neural network. Determining the second product profile may comprise training an artificial neural network using the first product profile, the first set of physiological effects, and the characteristic data regarding the plurality of subjects and providing the second set of physiological effects and the characteristic data regarding the target user to the artificial neural network.

In various embodiments, the first and second product profiles each contain at least one common compound identifier.

In various embodiments, the artificial neural network comprises a feedforward neural network, a radial basis function network, a self-organizing map, learning vector quantization, a recurrent neural network, a Hopfield network, a Boltzmann machine, an echo state network, long short term memory, a bi-directional recurrent neural network, a hierarchical recurrent neural network, a stochastic neural network, a modular neural network, an associative neural network, a deep neural network, a deep belief network, a convolutional neural networks, a convolutional deep belief network, a large memory storage and retrieval neural network, a deep Boltzmann machine, a deep stacking network, a tensor deep stacking network, a spike and slab restricted Boltzmann machine, a compound hierarchical-deep model, a deep coding network, a multilayer kernel machine, or a deep Q-network.

In various embodiments, the artificial neural network comprises a processor, a field-programmable gate array, an adaptive neuromorphic processor, a memory network, a long short-term memory, or a memristor.

In various embodiments, the method further comprises determining at least one association between one of the plurality of compound identifiers and one of the first set of physiological effects.

In various embodiments, the characteristic data comprise a genotypic profile, a metabolic profile, a proteomic profile, a lipomic profile, or a microbiomic profile.

In various embodiments, the characteristic data comprise a disease diagnosis. In some embodiments, the disease is Parkinson's disease, cancer, glaucoma, cannabis withdrawal syndrome, multiple sclerosis, or lupus.

In various embodiments, the characteristic data comprise a symptom. In some embodiments, the symptom comprises nausea, seizure, insomnia, lack of appetite, weight loss, depression, or narcolepsy.

In various embodiments, the first product profile further comprises a delivery route. In some embodiments, the delivery route comprises ingestion, inhalation, or transdermal administration.

In various embodiments, at least one of the plurality of compound identifiers identifies a isoprenoid or a terpene. In some embodiments, the isoprenoid is a cannabinoid.

In various embodiments, the method further comprises providing a survey to the plurality of subjects; receiving a plurality of survey responses from the plurality of subjects; and compiling the observational data regarding the plurality of subjects from the plurality of survey responses. In some embodiments, the survey includes a plurality of questions. In some embodiments, one of the plurality of questions is directed to a feeling of relaxation, nausea, alertness, well-being, or intoxication.

In various embodiments, the characteristic data comprise heart rate, tremor strength, or tremor frequency.

In various embodiments, the first product profile corresponds to a plant hybrid.

In various embodiments, at least one of the plurality of compound identifiers corresponds to a plant. In some embodiments, the at least one of the plurality of compound identifiers comprises taxonomic information of the plant, proteomic information of the plant, lipomic information of the plant, or genotypic information of the plant.

In various embodiments, the second product profile corresponds to a plant hybrid. In some embodiments, the plant hybrid is created by breeding or genetic modification.

In various embodiments, the characteristic data regarding the target user comprise a genotypic profile, a metabolic profile, a proteomic profile, a lipomic profile, or a microbiomic profile. In some embodiments, the characteristic data regarding the target user comprise a disease diagnosis. In some embodiments, the disease is Parkinson's disease, cancer, glaucoma, cannabis withdrawal syndrome, multiple sclerosis, or lupus.

In various embodiments, the characteristic data regarding the target user comprise a symptom. In some embodiments, the symptom comprises nausea, seizure, insomnia, lack of appetite, weight loss, depression, or narcolepsy.

In various embodiments, the method further comprises receiving the second set of physiological effects from a user via a user interface.

In various embodiments, consumption by each of the plurality of subjects comprises ingestion, inhalation, or transdermal administration.

In some embodiments, the first product profile corresponds to a plant extract. In some embodiments, the first product profile comprises supplemental information regarding the plant, the supplemental information comprising growth conditions, growth procedures, strain conditions, harvesting conditions, harvesting procedures, drying conditions, drying procedures, processing conditions, processing procedures, extraction conditions, extraction procedures, storage conditions, or storage procedures.

In various embodiments, reading observational data comprises reading sensor data from a sensor. In some embodiments, the sensor is a biometric sensor. In some embodiments, the sensor comprises an accelerometer, a camera, a microphone, a blood pressure sensor, an electroencephalograph, or a heart rate sensor. In some embodiments, the sensor is embedded in a wearable device. In some embodiments, the sensor is embedded in a smartphone.

In various embodiments, the method further comprises providing a cognitive test to the plurality of subjects; receiving a plurality of cognitive data from the plurality of subjects; and compiling the observational data regarding the plurality of subjects from the plurality of cognitive data.

In various embodiments, the method further comprises providing a game to the plurality of subjects; receiving a plurality of performance data from the plurality of subjects; and compiling the observational data regarding the plurality of subjects from the plurality of performance data.

### BRIEF DESCRIPTION OF THE SEVERAL VIEWS OF THE DRAWINGS

**Fig. 1** depicts a system according to embodiments of the present disclosure.
**Fig. 2** illustrates a method of training an artificial neural network according to embodiments of the present disclosure.
**Figs. 3A-B** depict inputs and outputs of an artificial neural network according to embodiments of the present disclosure.
**Fig. 4** illustrates a method of determining the physiological effects of a product according to embodiments of the present disclosure.
**Fig. 5** illustrates a method of providing a product recommendation according to embodiments of the present disclosure.
**Fig. 6** depicts a computing node according to an embodiment of the present invention.

### DETAILED DESCRIPTION

According to various embodiments of the present disclosure, systems, methods and computer program products are provided for distributed data gathering and recommendation in phytotherapy. By collecting data from users of various products and analyzing that data in light of the product profiles, recommendations are provided to users as to the most appropriate product for a desired effect.

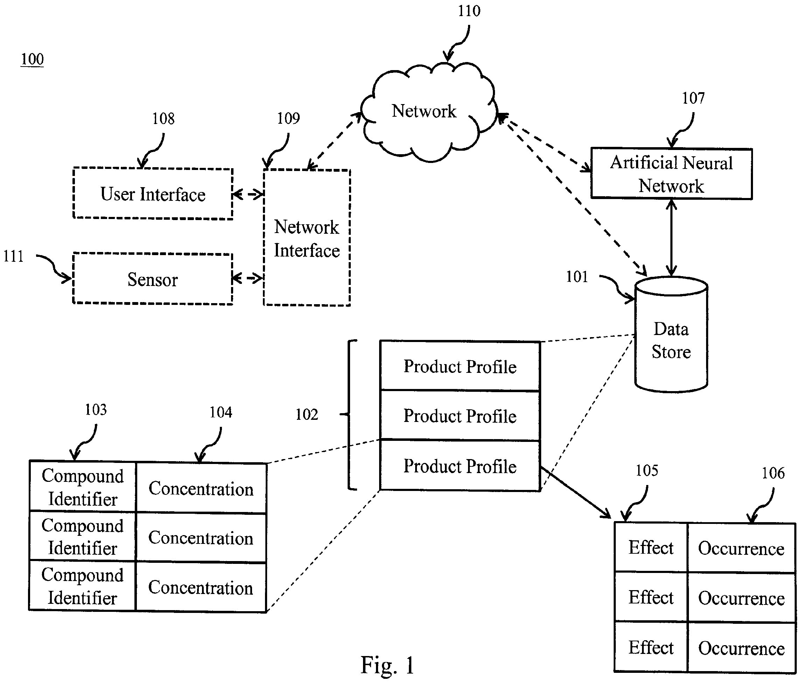

With reference now to **Fig. 1****,** a system **100** according to embodiments of the present disclosure is depicted. A data store **101** contains a plurality of product profiles **102.** In some embodiments, each product profile **102** includes at least one compound identifier **103** and a concentration **104.** In various embodiments, compound identifier **103** may be a chemical name (*e.g.,* tetrahydrocannabinol), chemical formula (*e.g.,* C₂₁H₃₀O₂), common name of a plant (*e.g.,* cannabis), plant species (*e.g., Cannabis indica*), or other identifying information of a plant strain (*e.g.*, Purple Kush). In various embodiments, concentration **104** is a measure of mass concentration, molar concentration, or volume concentration of the compound identified by compound identifier **103** within a product conforming to product profile **102.** In some embodiments, the product profile further includes a delivery route. The delivery route may include ingestion or inhalation. In some embodiments, product profile **102** further includes additional information regarding some or all of the included compounds. For example, where a compound identifier corresponds to a plant variety, supplemental information regarding its cultivation or handling may be included. Such information may include growth conditions and procedures (including, *e.g.,* strain conditions), harvesting conditions and procedures, drying conditions and procedures, processing conditions and procedures, extraction conditions and procedures, or storage conditions and procedures.

In some embodiments, each product profile **102** is further linked to at least one effect **105.** In some embodiments, effect **105** is associated with occurrence data **106.** In this way, the effects associated with a product conforming to product profile **102** are tracked. In some embodiments, effect **105** is determined from observational data regarding subjects that have consumed a product conforming with product profile **102.** In some embodiments, occurrence data **106** includes a percentage of users observing the associated effect. In some embodiments, occurrence data **106** includes characteristic data of subjects.

As set forth below, a product profile may serve both a descriptive and a prescriptive function. In particular, a product profile may describe a preexisting product being analyzed. A product profile may also be generated by the systems and methods provided for herein and prescribe a product to be made. In some cases, one or more constituents of a product profile may themselves correspond to a product profile. In this manner, the systems and methods of the present disclosure are suitable for analysis of products at various levels of granularity. Adopting the above example, a product profile corresponding to a plant strain might specify the percent by mass of tetrahydrocannabinol. A product profile corresponding to a consumable product containing that plant strain might specify the percent by mass of the plant strain. A product profile corresponding to an extract of that plant strain might specify the percent by mass of the plant strain in the extract.

In some embodiments, artificial neural network **107** is operatively connected to data store **101.** As set forth further below, product profile data, effect data, and data regarding subjects is fed into neural network **107** both to train neural network **107** and to provide recommendations and predictions regarding products. It will be appreciated that artificial neural network **107** may be resident on the same or different computing node as data store **101.** In addition, it will be appreciated that various types of artificial neural network are suitable for use according to embodiments of the present disclosure.

Suitable artificial neural networks include but are not limited to a feedforward neural network, a radial basis function network, a self-organizing map, learning vector quantization, a recurrent neural network, a Hopfield network, a Boltzmann machine, an echo state network, long short term memory, a bi-directional recurrent neural network, a hierarchical recurrent neural network, a stochastic neural network, a modular neural network, an associative neural network, a deep neural network, a deep belief network, a convolutional neural networks, a convolutional deep belief network, a large memory storage and retrieval neural network, a deep Boltzmann machine, a deep stacking network, a tensor deep stacking network, a spike and slab restricted Boltzmann machine, a compound hierarchical-deep model, a deep coding network, a multilayer kernel machine, or a deep Q-network.

In some embodiments, artificial neural network **107** is implemented in software. In some embodiments, artificial neural network **107** is implemented with custom hardware such as a processor, a field-programmable gate array, an adaptive neuromorphic processor, a memory network, a long short-term memory, or a memristor.

In some embodiments, user interface **108** is provided. User interface **108** may be a web-based user interface or a native user interface operating on a mobile device or a desktop device in various embodiments. In general, any computing node equipped with a display is suitable for user interface **108** according to the present disclosure. As set forth further below, user interface **108** is adapted to provide a survey to a user in order to collect both characteristic information regarding that user, and observational data regarding that user's experiences. In some embodiments, a user is queried through the user interface for effects experienced after consuming a product conforming to a product profile. Once effect data is collected, it is stored in data store **101.** In some embodiments, the raw data is stored in data store **101.** In some embodiments, aggregate data is stored pertaining to each product profile **102.**

User interface **108** may include a variety of tools for collection of user data. As noted above, in some embodiments, user interface **108** includes a survey. In some embodiments, user interface **108** includes a testing component, for example a cognitive test or challenge for a user to complete. For example, typing speed may be measured while a user completes a survey through user interface **108.** Additional exemplary tests include measuring the relative time to input a password, whether already existing in an external application, or established by the user for thius purpose. Such password tests may collect, for example, relative rate on input, and number of failures. In yet other embodiments, user interface **108** includes a game, a user's performance of which is suitable for determining cognitive ability or dexterity.

As noted above, user interface **108** may be displayed by any computing node having a display. In some embodiments, the computing node displaying user interface **108** communicates through a network interface **109** over a network **110.** In some embodiments, network **110** is the internet, however, network **110** may include a cellular network, a radio network, a LAN, a WAN, or any number of other data networks known in the art. It will be appreciated that user interface **108,** artificial neural network **107** and data store **101** may be spread across one or many computing nodes, which may be in communication with each other through network **110.**

In embodiments with user interface **108,** it is further adapted for a user to indicate a set of preferred effects and to receive from data store **101** or artificial neural network **107** a recommendation based on that set of preferred effects.

In some embodiments, one or more sensor **111** is provided. Sensor **111** may include an accelerometer, a camera, a microphone, a blood pressure sensor, a heart rate sensor, or other sensor known in the art. Such sensor **111** is configured to collect data from the user, either on an ongoing basis or solely at the time of user interaction with user interface **108.** In some embodiments, sensor **111** is embedded in a wearable device, such as an existing fitness tracker, smartphone, sensor equipped cane, augmented reality glasses, or other devices known in the art. In some embodiments, sensor **111** is coupled to the same computing node as hosts under interface **108.** In other embodiments, sensor **111** separately transmits data via network interface **109.** In exemplary embodiments, a camera is used to measure pupil dilation. In other exemplary embodiments, an accelerometer is used to measure shake or balance of a user. In yet other exemplary embodiments, a microphone is used to record speech, which is then analyzed for speed, slurring, or changes in speech pattern.

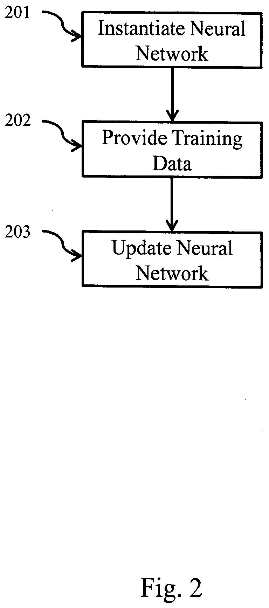

Referring now to **Fig. 2****,** a method of training a neural network according to embodiments of the present disclosure is illustrated. An artificial neural network is instantiated **201.** In some embodiments, the artificial neural network comprises a input layer, one or more hidden layers, and an output layer. However, it will be appreciated that various artificial neural network configurations are suitable for use according to the present disclosure. Training data are provided **202** to the artificial neural network. In some embodiments, training data include a product profile. A product profile corresponds to a product that may be consumed by a user. The product profile includes compound identifiers and corresponding concentrations. In this way, a product identifier provides the information necessary to characterize a product. As a simple example, a product identifier for bread might indicate 28% water by volume, 71% flour by volume, and 1% yeast by volume. In some embodiments, training data also include a set of physiological effects associated with the product profile.

In some embodiments, the physiological effects associated with the product profile are determined from observational data regarding a plurality of subjects. The observational data may include a user's assessment of relaxation, nausea, alertness, well-being, or intoxication. In some embodiments, the user's assessment is collected through a user interface. For example, a user may be queried as to a numeric rating for one or more of relaxation, nausea, alertness, well-being, or intoxication.

Based on the training data, the neural network is updated **203.** It will be appreciated that a variety of methods are suitable for each of the artificial neural networks according to the present disclosure. For example, back propagation may be used to train the neural network. In certain such embodiments, a product identifier is provided at the input layer of the neural network, and a result is determined from the output layer of the neural network. The physiological effects associated with the product profile are compared with the output of the neural network, and corrections are propagated back up through the hidden layers of the neural network to update it. However, in other embodiments, the input and outputs are reversed. Thus, the physiological effects are provided to the input layer as part of the training process while the product identifier is determined from the output layer. It will be appreciated that certain artificial neural networks are bidirectional, and thus input may be provided to the output layer with results being determined at the input layer.

In some embodiments, training data further include characteristic data for a user. In various embodiments, such characteristic data include a genotypic profile, a metabolic profile, a proteomic profile, a lipomic profile, or a microbiomic profile. In some embodiments, the characteristic data include a disease profile. In some such embodiments, the disease profile includes a diagnosis of a disease, which may include one or more of Parkinson's disease, cancer, glaucoma, cannabis withdrawal syndrome, multiple sclerosis, or lupus.

In some embodiments, training data include a delivery route. The delivery route may include ingestion, inhalation, or transdermal administration.

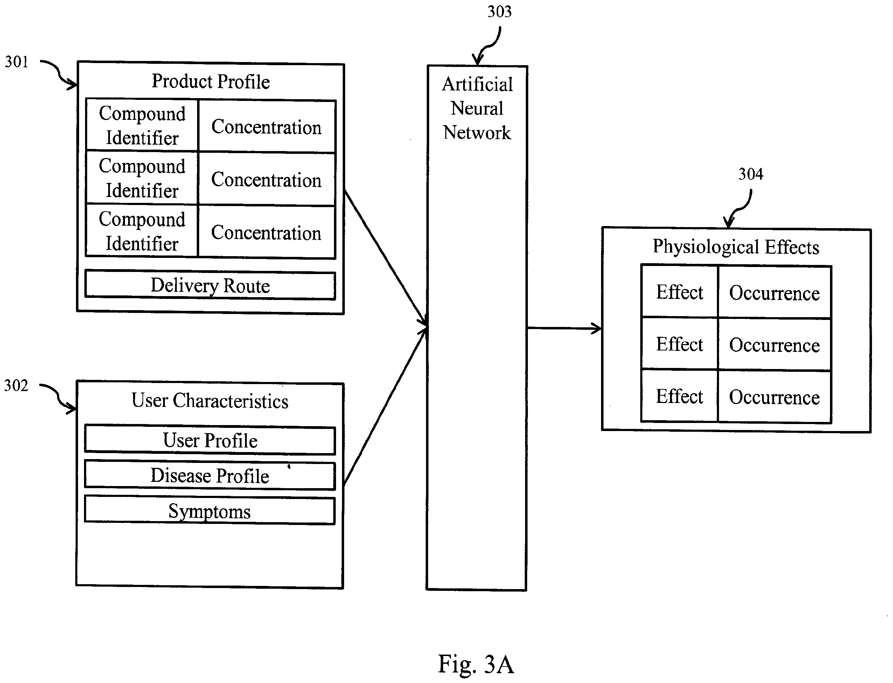

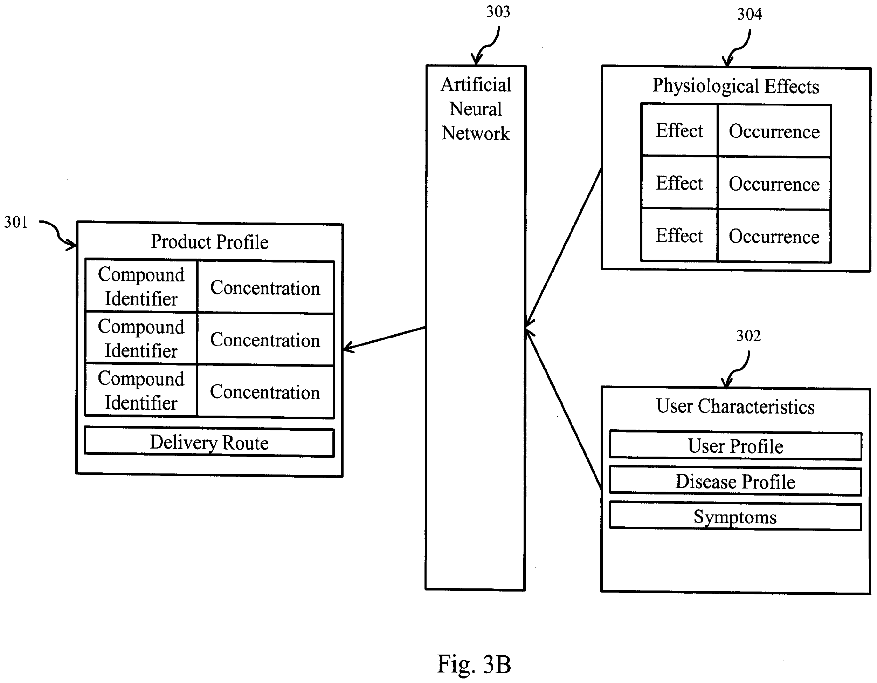

Referring now to **Figs. 3A-B****,** inputs and outputs of an artificial neural network are illustrated according to embodiments of the present disclosure. In **Fig. 3A**, a product profile **301** and optional user characteristics **302** are provided to trained artificial neural network **303.** Physiological effects **304** are obtained as output. The arrangement of **Fig. 3A** is suitable for determining a set of physiological effects **304** based on a product profile and optional user characteristics. For example, the effects of a previously unknown product can be predicted. In **Fig. 3B****,** physiological effects **304** and optional user characteristics **302** are provided to trained artificial neural network **303.** Product profile **301** is obtained as output. The arrangement of **Fig. 3B** is suitable for determining a product on the basis of desired physiological effects. For example, a previously unknown product can be generated that is likely to have the input effects. As discussed above, the training process entails applying known training data to neural network **303** with the same arrangement of inputs and outputs.

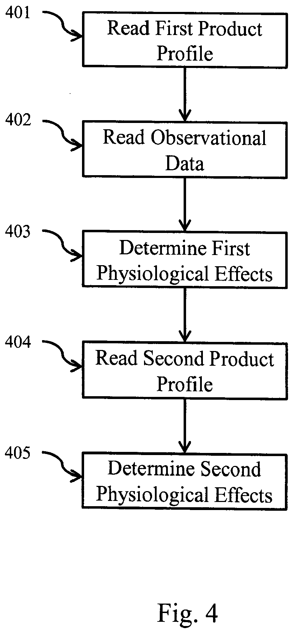

Referring now to **Fig. 4****,** a method of predicting the effects of a product according to embodiments of the present disclosure is illustrated. A first product profile is read **401.** As described above with reference to **Fig. 1****,** the product profile includes compound identifiers and associated concentrations. Observational data regarding subjects is read **402.** The observational data pertain to subjects that have consumed a product conforming with the first product profile. From the observational data, first physiological effects associated with the first product profile are determined **403.** In some embodiments, determining the physiological effects includes analyzing user responses to a survey. For example, users may be queried through a user interface to rate the occurrence of a plurality of effects after consuming a product conforming with the first product profile. In some embodiments, only the most frequently occurring effects are considered. In other embodiments, all reported effects are considered. In yet other embodiments, a determination is made as to statistical significance, and only statistically significant effects are considered.

A second product profile is read **404.** A second set of physiological effects is determined **405.** The second set of physiological effects is associated with the second product profile, and is determined based on the first product profile, the second product profile, and the first set of physiological effects. In some embodiments, this determination is made using an artificial neural network as described above. For example, this determination may be made by training an artificial neural network using the first product profile and the first set of physiological effects before providing the second profile to the artificial neural network to obtain the second set of physiological effects.

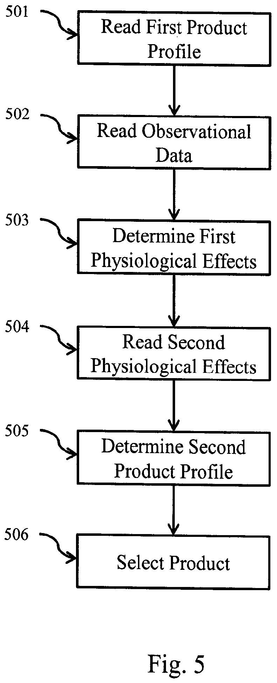

Referring now to **Fig. 5****,** a method of recommending a product according to embodiments of the present disclosure is illustrated. A first product profile is read **501.** As described above with reference to **Fig. 1****,** the product profile includes compound identifiers and associated concentrations. Observational data regarding subjects is read **502.** The observational data pertain to subjects that have consumed a product conforming with the first product profile. From the observational data, first physiological effects associated with the first product profile are determined **503.** In some embodiments, determining the physiological effects includes analyzing user responses to a survey. For example, users may be queried through a user interface to rate the occurrence of a plurality of effects after consuming a product conforming with the first product profile. In some embodiments, only the most frequently occurring effects are considered. In other embodiments, all reported effects are considered. In yet other embodiments, a determination is made as to statistical significance, and only statistically significant effects are considered.

A second set of physiological effects is read **504.** A second product profile is determined 505. The second product profile is associated with the second set of physiological effects, and is determined based on the first product profile, the first set of physiological effects, and the second set of physiological effects. In some embodiments, this determination is made using an artificial neural network as described above. In certain such embodiments, this determination is made by training an artificial neural network using the first product profile and the first set of physiological effects before providing the second set of physiological effects to the artificial neural network to obtain the second product profile.

In some embodiments, a product is selected **506** from a set of predetermined products based on the determined product profile. In some embodiments, a product is selected that substantially conforms with the product profile. However, in some embodiments, where a substantially conforming product is not available, the closest available product is selected.

In some embodiments, the product profiles correspond to plant varieties. For example, a product profile might indicate 94% *cannabis indica* and 6% *cannabis sativa,,* corresponding to a particular hybrid. In certain such embodiments, the second product profile is indicative of a hybrid that is predicted to have the input physiological effects. Such a hybrid may then be created. Creation of a hybrid may be through breeding or through genetic modification. It will be appreciated once an indicated hybrid has been consumed, the observed effects may be used to further refine artificial neural networks according to the present disclosure.

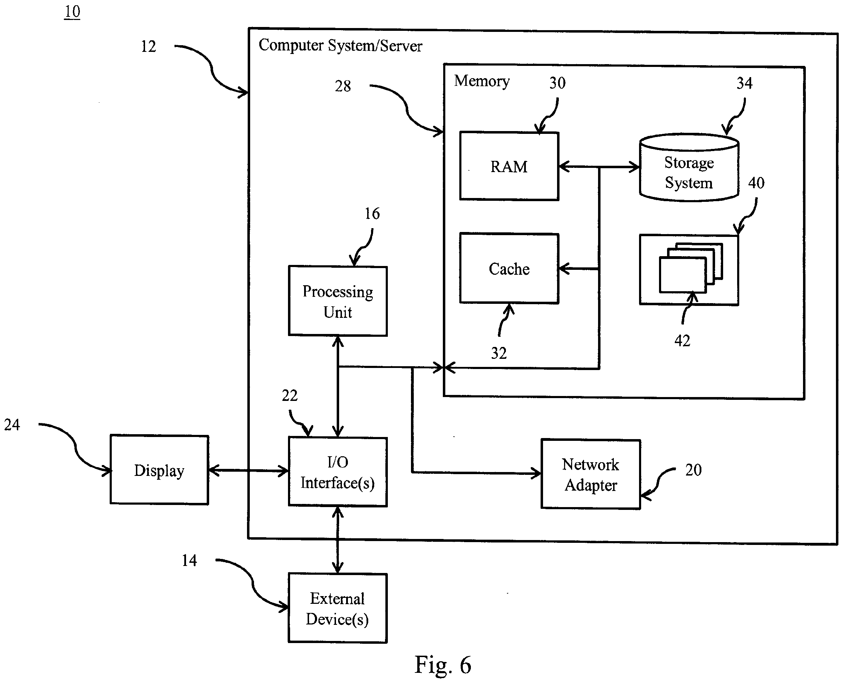

Referring now to **Fig. 6****,** a schematic of an example of a computing node is shown. Computing node **10** is only one example of a suitable computing node and is not intended to suggest any limitation as to the scope of use or functionality of embodiments of the invention described herein. Regardless, computing node **10** is capable of being implemented and/or performing any of the functionality set forth hereinabove.

In computing node **10** there is a computer system/server **12,** which is operational with numerous other general purpose or special purpose computing system environments or configurations. Examples of well-known computing systems, environments, and/or configurations that may be suitable for use with computer system/server **12** include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server **12** may be described in the general context of computer system-executable instructions, such as program modules, being executed by a computer system. Generally, program modules may include routines, programs, objects, components, logic, data structures, and so on that perform particular tasks or implement particular abstract data types. Computer system/server **12** may be practiced in distributed cloud computing environments where tasks are performed by remote processing devices that are linked through a communications network. In a distributed cloud computing environment, program modules may be located in both local and remote computer system storage media including memory storage devices.

As shown in **Fig. 1****,** computer system/server **12** in computing node **10** is shown in the form of a general-purpose computing device. The components of computer system/server **12** may include, but are not limited to, one or more processors or processing units **16,** a system memory **28,** and a bus **18** that couples various system components including system memory **28** to processor **16.**

Bus **18** represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus.

Computer system/server **12** typically includes a variety of computer system readable media. Such media may be any available media that is accessible by computer system/server **12,** and it includes both volatile and non-volatile media, removable and non-removable media.

System memory **28** can include computer system readable media in the form of volatile memory, such as random access memory (RAM) **30** and/or cache memory **32.** Computer system/server **12** may further include other removable/non-removable, volatile/non-volatile computer system storage media. By way of example only, storage system **34** can be provided for reading from and writing to a non-removable, non-volatile magnetic media (not shown and typically called a "hard drive"). Although not shown, a magnetic disk drive for reading from and writing to a removable, non-volatile magnetic disk (*e.g.,* a "floppy disk"), and an optical disk drive for reading from or writing to a removable, non-volatile optical disk such as a CD-ROM, DVD-ROM or other optical media can be provided. In such instances, each can be connected to bus **18** by one or more data media interfaces. As will be further depicted and described below, memory **28** may include at least one program product having a set (*e.g.,* at least one) of program modules that are configured to carry out the functions of embodiments of the invention.

Program/utility **40,** having a set (at least one) of program modules **42,** may be stored in memory **28** by way of example, and not limitation, as well as an operating system, one or more application programs, other program modules, and program data. Each of the operating system, one or more application programs, other program modules, and program data or some combination thereof, may include an implementation of a networking environment. Program modules **42** generally carry out the functions and/or methodologies of embodiments of the invention as described herein.

Computer system/server **12** may also communicate with one or more external devices **14** such as a keyboard, a pointing device, a display **24,** etc.; one or more devices that enable a user to interact with computer system/server **12;** and/or any devices (*e.g*., network card, modem, etc.) that enable computer system/server **12** to communicate with one or more other computing devices. Such communication can occur via Input/Output (I/O) interfaces **22.** Still yet, computer system/server **12** can communicate with one or more networks such as a local area network (LAN), a general wide area network (WAN), and/or a public network (*e.g*., the Internet) via network adapter **20.** As depicted, network adapter **20** communicates with the other components of computer system/server **12** via bus **18.** It should be understood that although not shown, other hardware and/or software components could be used in conjunction with computer system/server **12.** Examples, include, but are not limited to: microcode, device drivers, redundant processing units, external disk drive arrays, RAID systems, tape drives, and data archival storage systems, etc.

The embodiments disclosed herein may be implemented as a system, a method, and/or a computer program product. The computer program product may include a computer-readable storage medium (or media) having computer-readable program instructions thereon for causing a processor to carry out aspects of the present invention.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (*e.g*., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer-readable program instructions for storage in a computer-readable storage medium within the respective computing/processing device.

Computer-readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object-oriented programming language such as Smalltalk, C++ or the like, and conventional procedural programming languages, such as the "C" programming language or similar programming languages. The computer-readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A method comprising:
reading a first product profile, the first product profile comprising a plurality of compound identifiers and a plurality of concentrations, each of the plurality of compound identifiers identifying a compound and each of the plurality of concentrations being associated with one of the plurality of compound identifiers;
reading observational data regarding a plurality of subjects, each of the plurality of subjects having consumed a first product substantially conforming with the first product profile;
determining from the observational data a first set of physiological effects associated with the first product profile.

2. The method of claim 1, further comprising:
reading a second product profile;
determining a second set of physiological effects associated with the second product profile based on the first product profile, the second product profile, and the first set of physiological effects.

3. The method of claim 1, further comprising:
reading a second set of physiological effects;
determining a second product profile associated with the second set of physiological effects based on the first product profile, the first set of physiological effects, and the second set of physiological effects.

4. The method of claim 2, wherein determining the second set of physiological effects comprises:
training an artificial neural network using the first product profile and the first set of physiological effects;
providing the second product profile to the artificial neural network.

5. The method of claim 3, wherein determining the second product profile comprises:
training an artificial neural network using the first product profile and the first set of physiological effects;
providing the second set of physiological effects to the artificial neural network.

6. The method of claim 1, further comprising:
reading characteristic data regarding the plurality of subjects;
reading a second product profile;
reading characteristic data regarding a target user;
determining a second set of physiological effects associated with the second product profile based on the first product profile, the second product profile, the first set of physiological effects, and the characteristic data regarding the target user.

7. The method of claim 1, further comprising:
reading characteristic data regarding the plurality of subjects;
reading a second set of physiological effects;
reading characteristic data regarding a target user;
determining a second product profile associated with the second set of physiological effects based on the first product profile, the first set of physiological effects, the second set of physiological effects, and the characteristic data regarding the target user.

8. The method of claim 6, wherein determining the second set of physiological effects comprises:
training an artificial neural network using the first product profile, the first set of physiological effects, and the characteristic data regarding the plurality of subjects;
providing the second product profile and the characteristic data regarding the target user to the artificial neural network.

9. The method of claim 7, wherein determining the second product profile comprises:
training an artificial neural network using the first product profile, the first set of physiological effects, and the characteristic data regarding the plurality of subjects;
providing the second set of physiological effects and the characteristic data regarding the target user to the artificial neural network.

10. The method of claim 6 or 7, wherein the characteristic data comprise a genotypic profile, a metabolic profile, a proteomic profile, a lipomic profile, a microbiomic profile, a disease diagnosis, or a symptom.

11. The method of any of claim 1-10, wherein at least one of the plurality of compound identifiers identifies a isoprenoid, a terpene, or a cannabinoid.

12. The method of any of claims 1-10, wherein the first product profile or the second product profile corresponds to a plant hybrid or a plant extract.

13. The method of any of claims 1-10, wherein at least one of the plurality of compound identifiers corresponds to a plant, and wherein the at least one of the plurality of compound identifiers comprises taxonomic information of the plant, proteomic information of the plant, lipomic information of the plant, or genotypic information of the plant.

14. The method of claim 13, wherein the first product profile comprises supplemental information regarding the plant, the supplemental information comprising growth conditions, growth procedures, strain conditions, harvesting conditions, harvesting procedures, drying conditions, drying procedures, processing conditions, processing procedures, extraction conditions, extraction procedures, storage conditions, or storage procedures.

15. The method of any of claims 1-10, further comprising:
compiling the observational data regarding the plurality of subjects by administration of a cognitive test, a game, or a survey, or by reading sensor data from a biometric sensor.
